# EUROPEAN PATENT APPLICATION

(11) **EP 4 311 444 A1**
(43) Date of publication of application: **31.01.2024**
(21) Application number: 22803155.5
(22) Date of filing: 30.05.2022
(51) Int. Cl.: A24F 40/46, H05B 3/03, H05B 3/06, A24F 40/44, A24F 40/10

(54) **ATOMIZER AND ATOMIZATION ASSEMBLY**

(71) Applicant: Shenzhen Huachengda Precision Industry Co., Ltd., Shenzhen, Guangdong 518000 (CN)
(72) Inventor: CHEN, Ping, Shenzhen, Guangdong 518000 (CN)
(74) Representative: Westphal, Mussgnug & Partner, Patentanwälte mbB
(86) International application number: PCT/CN2022/095970
(87) International publication number: WO 2023/230761

(57) **Abstract**

The present invention discloses an atomizer and an atomizing assembly, including an adsorption substrate, a heating member, and a lead. The adsorption substrate is configured to adsorb a liquid atomizable medium, the heating member includes a heating body and an electrode connected with the heating body, and the heating body is disposed on a sidewall of the adsorption substrate. The electrode is partially or completely embedded in the adsorption substrate, and the lead is electrically connected with a portion of the electrode that is located in the adsorption substrate. Since the electrode extending from the heating body is partially or completely embedded in the adsorption substrate, and one end of the lead is embedded in the adsorption substrate, the adsorption substrate wraps the electrode and part of the lead of the heating member; due to that the heating body is poor in strength and easy to be deformed, while the structural strength of the electrode is good, the electrode can be embedded in the porous ceramic, so that the heating body of the heating member is not prone to being deformed due to the pulling of the lead during the assembly process, thereby avoiding the problem of core overburning because of the separation of the heating member and the adsorption substrate.

## Description

### FIELD

The present invention relates to the field of atomization, and more specifically, to an atomizer and an atomizing assembly.

### BACKGROUND

The electronic atomization technology is a technology that generates atomized steam by electrically heating the liquid to its boiling point to evaporate the liquid, and currently is mainly used in the field of electronic cigarettes.

With the innovation and the development of the atomization technology, the electronic atomization technology has also been used in more other fields, such as the fields of beauty and medical treatment. A major component of a resistive atomizer is an atomizing core, which is mainly composed of a heating member and a liquid conducting material. The heating member formed by curling a flat metal is widely used due to the advantages of easy temperature adjustment, controllable heat, uniform spacing, easy batch production, low cost, etc.

However, currently, such heating member is mostly used in the field of liquid conducting cotton, and has not been widely used in the structure of atomized core composed of porous ceramics. The main reason is that after the heating member is curled and formed, it is integrally sintered with the porous ceramic slurry through in-mold forming, the heating member is easily separated from the porous ceramic, resulting in the problem of core overburning, which causes that such heating member has not been popularized in a large batch.

### SUMMARY

A technical problem to be solved by the present invention is, in view of the above defects in the prior art, to provide an atomizer and an atomizing assembly.

A technical solution adopted by the present invention to solve the technical problem is to provide an atomizing assembly, including an adsorption substrate, a heating member, and a lead;
wherein the adsorption substrate is configured to adsorb a liquid atomizable medium,
wherein the heating member includes a heating body and an electrode connected with the heating body,
wherein the heating body is disposed on a sidewall of the adsorption substrate, and the electrode is partially or completely embedded in the adsorption substrate; and
wherein the lead is electrically connected with a portion of the electrode that is located in the adsorption substrate.

In some embodiments, the adsorption substrate is a column having a through hole in the middle, and the heating body is located on a sidewall of the through hole or an outer wall of the adsorption substrate.

In some embodiments, the heating body is curled in a circumferential direction, and the heating body is embedded on the inner wall of the through hole or the outer wall of the adsorption substrate.

In some embodiments, the through hole is circular, runway or U-shaped.

In some embodiments, the heating body is in a mesh shape and/or a grid shape.

In some embodiments, the adsorption substrate is a porous ceramic or a porous glass.

In some embodiments, the electrode is flat or bent.

In some embodiments, the electrode is provided with an embedded position configured for the embedding of the adsorption substrate.

In some embodiments, the embedded position is a hole or a slot, and the lead is led out from a middle of an end surface of the sidewall of the adsorption substrate.

The present invention provides an atomizer, including the atomizing assembly of any one of the above.

Implementation of the atomizer and the atomizing assembly of present invention provides the following beneficial effects: since the electrode extending from the heating body is partially or completely embedded in the adsorption substrate, and one end of the lead is embedded in the adsorption substrate, the adsorption substrate wraps the electrode and part of the lead of the heating member; due to that the heating body is poor in strength and easy to be deformed, while the structural strength of the electrode is good, the electrode can be embedded in the porous ceramic, so that the heating body of the heating member is not prone to being deformed due to the pulling of the lead during the assembly process, thereby avoiding the problem of core overburning because of the separation of the heating member and the adsorption substrate.

### BRIEF DESCRIPTION OF THE DRAWINGS

Subject matter of the present invention will be described in even greater detail below based on the exemplary figures. In the accompanying drawings:
Fig. 1 is a three-dimensional structural diagram of an atomizing assembly in an embodiment of the present invention;
Fig. 2 is an exploded diagram of the atomizing assembly in Fig. 1;
Fig. 3 is a schematic diagram showing the connection of the unfolded heating member and the leads in Fig. 2;
Fig. 4 is a schematic diagram when the embedded position in Fig. 3 is a slot;
Fig. 5 is a sectional view of an atomizing assembly with a heating member having a U-shaped cross-section embedded in an adsorption substrate;
Fig. 6 is schematic diagram showing the connection between the formed heating member in Fig. 5 and the leads;
Fig. 7 is a sectional view of the atomizing assembly when the cross-section of the heating member is U-shaped and the through hole of the adsorption substrate is runway shaped;
Fig. 8 is a sectional view of the atomizing assembly when the cross-section of the heating member is U-shaped, the through hole of the adsorption substrate is runway shaped, and the shape of the adsorption substrate is square;
Fig. 9 is a sectional diagram of the atomizing assembly when the cross-section of the heating member is C-shaped;
Fig. 10 is a schematic diagram of an end surface when the heating member in Fig. 9 is connected with the leads;
Fig. 11 is a sectional diagram of the atomizing assembly when the cross-section of the heating member is semicircular; and
Fig. 12 is a schematic diagram of an end surface when the heating member in Fig. 11 is connected with the leads.

### DETAILED DESCRIPTION

For better understanding of the technical features, objects and effects of the present invention, the specific embodiments of the present invention will be described in detail with reference to the accompanying drawings.

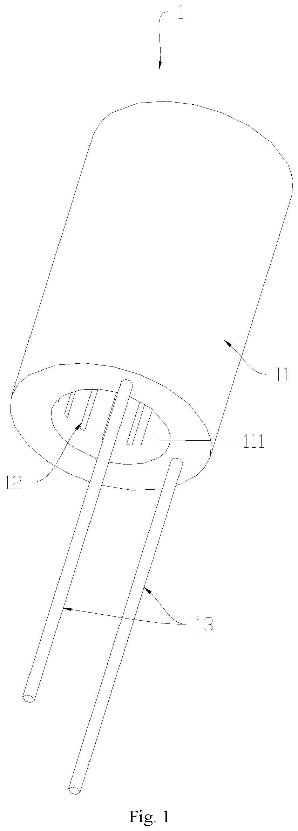

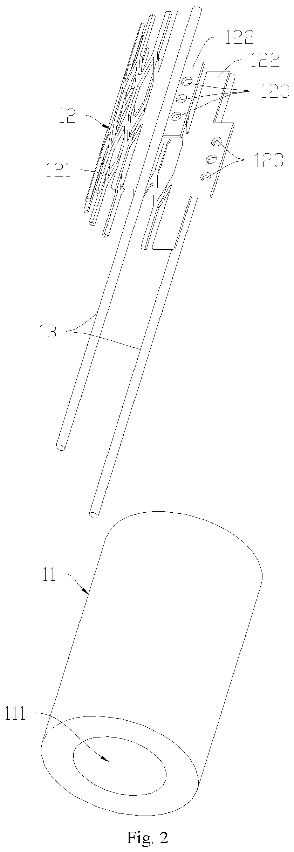

As shown in Fig. 1 and Fig. 2, an atomizing device in a preferred embodiment of the present invention includes an atomizer and a power supply device. The atomizer includes an atomizing assembly 1, and the atomizing assembly 1 includes an adsorption substrate 11, a heating member 12, and a lead 13.

The adsorption substrate 11 is configured to adsorb a liquid atomizable medium. In this embodiment, the adsorption substrate 11 is a porous ceramic 112 and is formed by sintering, and utilizes the porous characteristics of the ceramic to adsorb the liquid atomizable medium. In other embodiments, the adsorption substrate 11 may also be other hard material that has adsorption holes and can adsorb the liquid atomization medium, such as a porous glass.

As shown in Fig. 2 to Fig. 6, in some embodiments, the heating member 12 includes a heating body 121 and an electrode 122 connected with the heating body 121. Furthermore, the heating body 121 is embedded on a sidewall of the adsorption substrate 11, and the electrode 122 is partially embedded in the adsorption substrate 11, so as to improve the adhesion between the heating member 12 and the adsorption substrate 11. Of course, the electrodes 122 may also be completely embedded in the adsorption substrate 11.

The lead 13 is electrically connected with the portion of the electrode 122 that is located in the adsorption substrate 11, so that the end of the lead 13 that is connected with the electrode 122 is buried in the adsorption substrate 11, and the other end of the lead 13 is electrically connected with the power supply device, to supply power for the heating member 12. When the heating member 12 is powered on to generate heat, the liquid atomizable medium in the adsorption substrate 11 is heated and atomized into aerosol, which flows out.

Since the electrode 122 extending from the heating body 121 is partially or completely embedded in the adsorption substrate 11, and one end of the lead 13 is embedded in the adsorption substrate 11, it is equivalent to using the adsorption substrate 11 to wrap the electrode 122 and part of the lead 13 of the heating member 12. Due to the structural limitation of the heating member 12, the heating body 121 is mostly composed of thinner lines and thus has a poor strength and is easy to be deformed, the electrode 122 itself is only for welding the lead 13 and has a good structural strength, therefore, the electrode 122 may be designed into various shapes, and the electrode 122 is partially or completely embedded in the porous ceramic 112. Since the electrode 122 of heating member 12 is wrapped by the hard porous ceramic 112, the heating body 121 of the heating member 12 is not prone to being deformed due to the pulling of the lead 13 during the assembly process, thereby avoiding the problem of core overburning because of the separation of the heating member 12 and the adsorption substrate 11.

In some embodiments, the adsorption substrate 11 is a column having a through hole 111 in the middle, and the heating body 121 is located on the sidewall surface of the through hole 111. In other embodiments, the heating body 121 may also be located on the outer wall surface of the adsorption substrate 11.

Preferably, the heating body 121 is curled in the circumferential direction. In this embodiment, the heating body 121 is embedded on the inner wall surface of the through hole 111, allowing the heating body 121 to uniformly heat and atomize the liquid atomizable medium on the surface of the adsorption substrate 11. When the heating body 121 is located on the outer wall surface of the adsorption substrate 11, it is embedded on the outer wall surface of the adsorption substrate 11.

The porous ceramic 112 has a columnar structure, the heating body 121 is curled formed, the heating body 121 of the heating member 12 is embedded in the inner wall of the porous ceramic 112, the electrode 122 is completely or mostly embedded in the porous ceramic 112, one end of the lead 13 is welded to the electrode 122 of the heating member 12 and embedded in the porous ceramic 112, the other end of the lead 13 extends out from the end surface of the porous ceramic 112, and the lead 13 is led out from the middle of the end surface of the sidewall of the porous ceramic 112, that is, the lead 13 extends out from the approximately central position of the wall thickness of the end surface of the sidewall, thereby improving the stability of the lead 13 in the adsorption substrate 11.

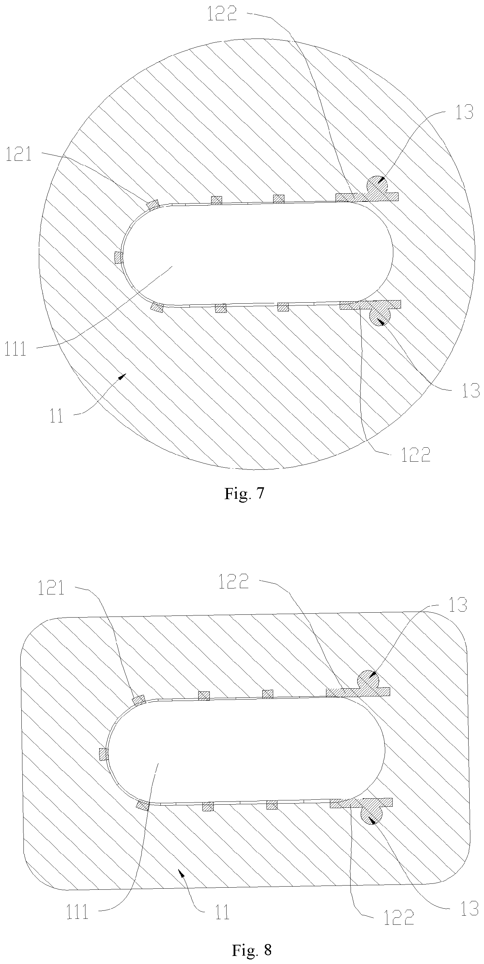

Of course, as shown in Fig. 7 and Fig. 8, in other embodiments, the outline shape of the adsorption substrate 11 may also be circular or square. In addition, the shape of the through hole 111 of the porous ceramic 112 is not limited to circular, but may also be runway shaped, U-shaped, C-shaped or other shape. The heating body 121 may be U-shaped or C-shaped corresponding to the shape of the inner wall of the through hole 111.

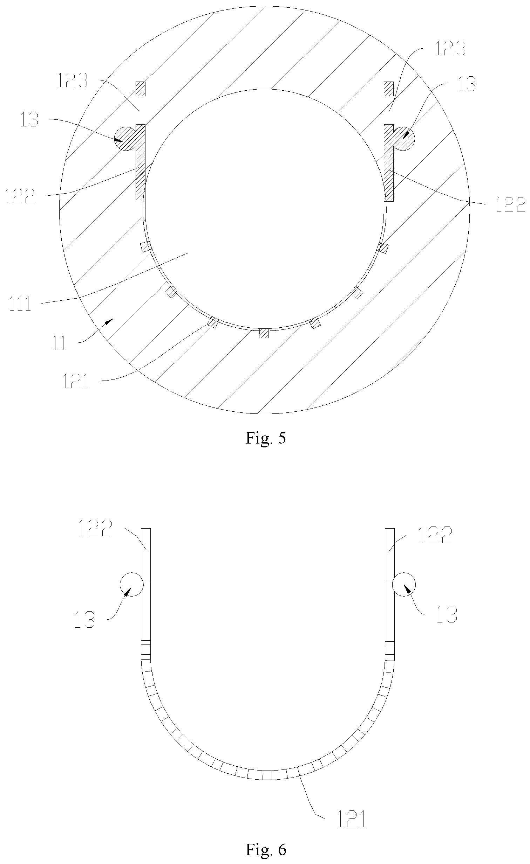

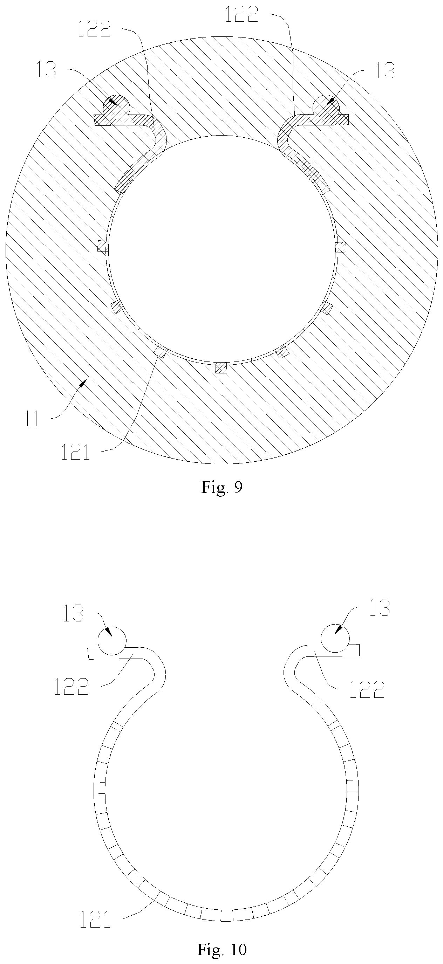

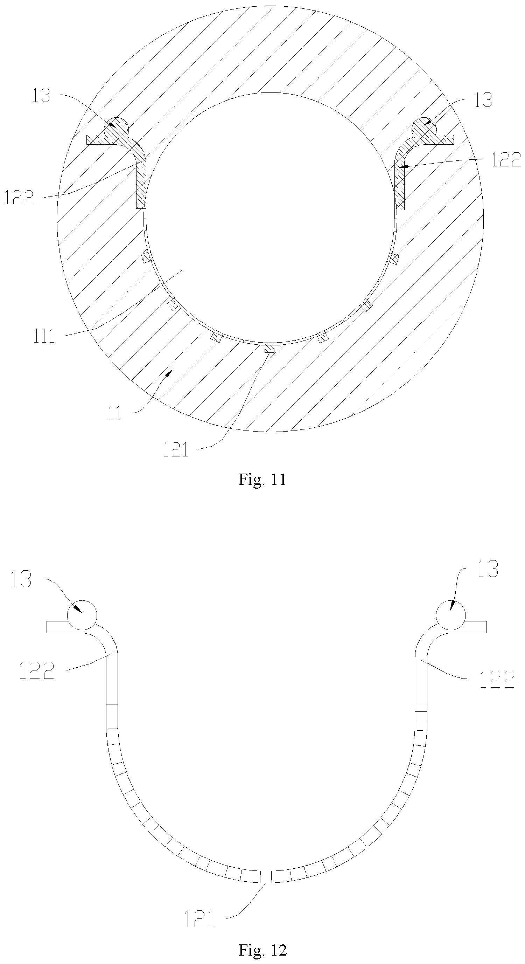

Generally, combined with Fig. 5, Fig. 6, and Fig. 9 to Fig. 12, in this embodiment, the flat heating member 12 may be wound after stamping, and the heating body 121 forms an arc shape, such as a semicircle shape or a C-shape, consistent with the shape of the inner wall of the through hole 111 of the adsorption substrate 11, and the electrode 122 is partially or completely embedded in the adsorption substrate 11. After the adsorption substrate 11 is formed, the stress from the lead 13 when assembling will not cause the core overburning caused by the separation of the heating body 121 and the adsorption substrate 11, which greatly improves the reliability of the atomizing assembly 1.

Preferably, the heating body 121 has a mesh shape, or a grid shape, or a combination shape of the two, which can enable the heating body 121 to heat the liquid atomizable medium on the adsorption substrate 11 more evenly, so that the liquid atomizable medium can flow stably to the heating body 121 and be heated, and meanwhile, the atomization speed is reduced, and the dry burning is avoided.

In some embodiments, the heating body 121 is embedded on the surface of the adsorption substrate 11, so that the heating body 121 can fully contact the adsorption substrate 11, and the core overburning problem caused by the separation of the heating body 121 and the adsorption substrate 11 will not occur.

It can be understood that the electrode 122 is flat or bent, so as to enhance the adhesion after being embedded into the adsorption substrate 11, and prevent from leaving the adsorption substrate 11.

Further, in order to improve the stability, the electrode 122 is provided with an embedded position 123 configured for the adsorption substrate 11 to be embedded during molding. After the adsorption substrate 11 is formed, the portion of the adsorption substrate 11 embedded in the embedded position 123 is engaged with the embedded position 123, which is not easy to be loosened or separated.

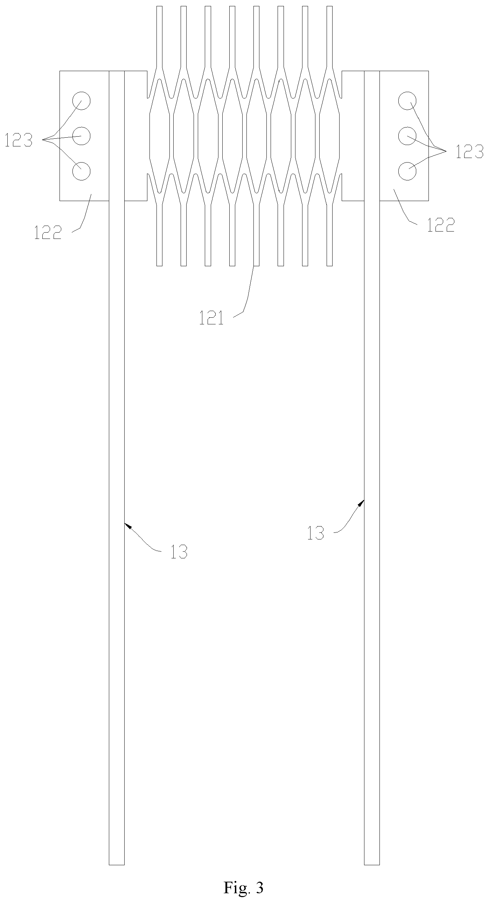

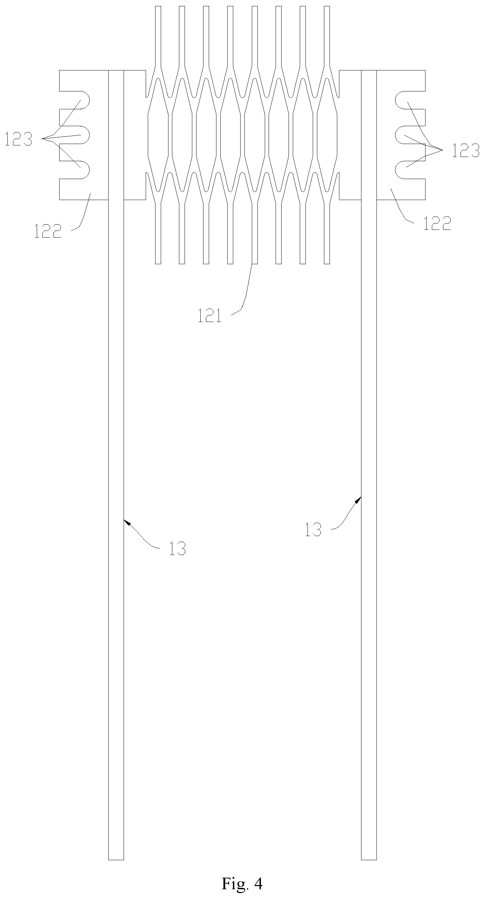

In some embodiments, the embedded positions 123 are holes as shown in Fig. 3, or the embedded position 123 may also be slots as shown in Fig. 4, or may include both holes and slots, wherein the numbers of the holes and the slots are not limited. The function of the embedded position 123 on the electrode 122 is to make the electrode 122 better embedded in the porous ceramic 112, so that the combination of the electrode 122 and the adsorption substrate 11 is more stable, thus making the lead 13 more stressed.

Preferably, the electrode 122 of the heating member 12 may adopt a plane structure, which is conducive to the welding of the lead 13. The electrode 122 may be bent relative to the heating body 121, and the bending angle is not limited. The heating member 12 may be formed by one-time stamping through a mold, and then placed in a mold after being welded with the lead 13, and then the porous ceramic 112 is formed by sintering after the ceramic slurry is injected, to cover part or all of the electrode 122.

Of course, in other embodiments, the heating member 12 in a planar shape may also be manufactured first, then welded with the lead 13, and then the heating member 12 is bent through a mold and/or a tool.

It is understood that the above-mentioned technical features can be used in any combination without restriction.

The disclosure described above of the present invention is illustrative but not restrictive scope of the present invention. Any equivalent structure, or equivalent process transformation, or directly or indirectly usage in other related technical field, all those be made in the same way are included within the protection scope of the present invention.

## Claims

1. An atomizing assembly, comprising an adsorption substrate (11), a heating member (12), and a lead (13);
wherein the adsorption substrate (11) is configured to adsorb a liquid atomizable medium,
wherein the heating member (12) comprises a heating body (121) and an electrode (122) connected with the heating body (121),
wherein the heating body (121) is disposed on a sidewall of the adsorption substrate (11), and the electrode (122) is partially or completely embedded in the adsorption substrate (11); and
wherein the lead (13) is electrically connected with a portion of the electrode (122) that is located in the adsorption substrate (11).

2. The atomizing assembly of claim 1, wherein the adsorption substrate (11) is a column having a through hole (111) in the middle, and the heating body (121) is located on a sidewall of the through hole (111) or an outer wall of the adsorption substrate (11).

3. The atomizing assembly of claim 2, wherein the heating body (121) is curled in a circumferential direction, and the heating body (121) is embedded on the inner wall of the through hole (111) or the outer wall of the adsorption substrate (11).

4. The atomizing assembly of claim 2, wherein the through hole (111) is circular, runway or U-shaped.

5. The atomizing assembly of any one of claims 1 to 4, wherein the heating body (121) is in a mesh shape and/or a grid shape.

6. The atomizing assembly of claim 5, wherein the adsorption substrate (11) is a porous ceramic (112) or a porous glass.

7. The atomizing assembly of any one of claims 1 to 4, wherein the electrode (122) is flat or bent.

8. The atomizing assembly of claim 7, wherein the electrode (122) is provided with an embedded position (123) configured for the embedding of the adsorption substrate (11).

9. The atomizing assembly of claim 8, wherein the embedded position (123) is a hole or a slot, and the lead (13) is led out from a middle of an end surface of the sidewall of the adsorption substrate (11).

10. An atomizer, comprising the atomizing assembly (1) of any one of claims 1 to 9.
